Europäisches Patentamt

European Patent Office

Office européen des brevets

(19)

(11) Veröffentlichungsnummer: **0 201 571**

**B1**

(12) **EUROPÄISCHE PATENTSCHRIFT**

(45) Veröffentlichungstag der Patentschrift:
**19.04.89**

(21) Anmeldenummer: **85905762.2**

(22) Anmeldetag: **30.10.85**

(86) Internationale Anmeldenummer:
**PCT/DE 85/00434**

(87) Internationale Veröffentlichungsnummer:
**WO 86/02840 (22.05.86 Gazette 86/11)**

(51) Int. Cl.⁴: **A 61 K  45/06,** A 61 K  31/40, A 61 K  31/275

(54) **PHARMAZEUTISCHE ZUSAMMENSETZUNG, IHRE HERSTELLUNG UND VERWENDUNG.**

(30) Priorität: **06.11.84  DE 3440881**

(43) Veröffentlichungstag der Anmeldung:
**20.11.86 Patentblatt 86/47**

(45) Bekanntmachung des Hinweises auf die Patenterteilung:
**19.04.89 Patentblatt 89/16**

(84) Benannte Vertragsstaaten:
**AT BE CH DE FR GB IT LI LU NL SE**

(56) Entgegenhaltungen:
**DE-A-3 115 993**
**DE-B-1 154 810**
**DE-B-1 158 083**

**Rote Liste, 1980, Editio Cantor, Aulendorf/Württ. (DE) see abstract 09023B, "Cordichin"**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **LOGE, Olaf, Bekassinenweg 37, D-1000 Berlin 27 (DE)**
Erfinder: **DOROW, Peter, Besingweg 15, D-1000 Berlin 22 (DE)**

## Beschreibung

Die Erfindung betrifft eine neue synergistisch wirkende Arzneimittelwirkstoff-Kombination, die aus dem Calcium-Antagonisten Verapamil und aus einem β-selektiven Sympathicomimetikum besteht.

Die erfindungsgemäße Kombination ist zur Behandlung des Asthma bronchiale geeignet.

Calcium-Antagonisten werden in der Medizin bekanntermaßen zur Behandlung von Herzrhythmusstörungen, cardialen Durchblutungsstörungen und zum Teil auch bei leichten bis mittelschweren Fällen des Bluthochdrucks verwendet.

Verapamil, dessen chemische Bezeichnung (5[(3.4-Dimethoxyphenethyl)-methylamino]-2-(3.4-dimethoxyphenyl)-2-isopropyl-valeronitril) ist, ist ein seit langem bekannter Calciumantagonist (DE-PS-1 154 810 und 1 158 083), der in der Medizin bei Angina pectoris und bei Herzrhythmusstörungen eingesetzt wird.

Die β-selektiven Sympathikomimetika, die auch β-Rezeptoren-Stimulatoren genannt werden, sind bekannte Bronchospasmolytika mit einer breiten Anwendung in Klinik und ärztlicher Praxis. Genannt seien beispielsweise Salbutamol (US-P-3 642 896 und 3 705 233), Fenoterol (DE-PS-1 286 047), Terbutalin (GB-PS-1 199 630), Reproterol (DE-PS-1 545 725 und 1 795 573), Hexoprenalin (DE-PS-1 215 729), Isoprenalin (US-P-2 308 232), Orciprenalin (DE-PS-1 275 069) und Clenbuterol (DE-AS-2 354 959) sowie der in der Entwicklung befindliche 4-(2-tert.-Butylamino-1-hydroxyethyl)-7-hydroxy-2-indolcarbonsäuremethylester (DE-OS-3 115 993).

Die bekannten β-Rezeptoren-Stimulatoren haben jedoch den Nachteil, daß sie in ihrer Eigenschaft als Substanzen mit β-adrenerger Wirkung sowohl tachycarde als auch bei Überdosis cardiotoxische Effekte haben.

Auf der Suche nach Möglichkeiten, diese unerwünschten Effekte von β-Rezeptor-Stimulatoren bei ihrer Anwendung zur Asthmabehandlung zu vermeiden, ist auch schon die Kombination eines β-Rezeptor-Stimulators mit einem Calcium-Antagonisten versucht worden. Die Untersuchungen von Shirichi Tomioka et al., (Jap. J. Allergology 31, 12, 1982) hatten ergeben, daß der Calcium-Antagonist Nifedipin zwar die in einer Placebo-Gruppe beobachtete Verschlechterung von Lungenfunktionsparametern milderte, jedoch in Kombination mit dem β$_2$-Rezeptor-Stimulator Terbutalin keinen therapeutischen Vorteil gegenüber einer Behandlung mit Terbutalin allein erbrachte und sogar die Herzfrequenz steigerte.

Es wurde nun überraschenderweise gefunden, daß der Calcium-Antagonist Verapamil in Kombination mit einem β-Rezeptor-Stimulator die bronchodilatatorische Wirkung verstärkt und darüber hinaus die Herzfrequenz gesenkt wird.

Hierzu wurden sowohl tierpharmakologische als auch klinische Untersuchungen durchgeführt.

1. Untersuchungen zur bronchospasmolytischen Wirkung an Meerschweinchen.

Bei Meerschweinchen wird durch Vernebeln einer Histaminhaltigen Aerosollösung eine Bronchokonstriktion ausgelöst, die durch einen Atemnotanfall sichtbar wird. Durch Vorbehandlung der Tiere mit bronchospasmolytisch wirkenden Substanzen in Aerosol-Form kann das Zeitintervall bis zum Auftreten Histamin-induzierter Atemnotanfälle (Bronchospasmen) verlängert werden. Die Meerschweinchen wurden mit Aerosolen vorbehandelt, die entweder einen β-Rezeptor-Stimulator allein, den Calciumantagonisten Verapamil allein oder eine Kombination beider Wirkstoffe enthielten. Als β-Rezeptor-Stimulator wurde in einem Test der 4-(2-tert.-Butylamino-1-hydroxyethyl)-7-hydroxy-2-indolcarbonsäure-methylester (RSH) verwendet.

Die Ergebnisse sind in der folgenden Tabelle I zusammengefaßt.

## Tabelle I

| | Zeit bis zum Auftreten des Atemnotfalls in Sekunden | | % Zeitverlängerung gegenüber der Kontrolle |
|---|---|---|---|
| | $\bar{x}$ | S$\bar{x}$ | |
| Kontrolle | 77,90 | 2,33 | |
| RSH 1 µg/ml Aerosol | 98,50 | 4,22 | 26,44 |
| Verapamil 1 mg/ml Aerosol | 96,10 | 3,67 | 23,36 |
| RSH 1 µg/ml und Verapamil 1 mg/ml | 127,20 | 3,65 | 63,29* |

*signifikant verschieden von der Kontrollgruppe, p <0,05

Die Zeitintervalle der nur mit RSH oder allein mit Verapamil vorbehandelten Meerschweinchen waren in der gewählten Dosierung nur geringfügig und nicht signifikant gegenüber dem Zeitintervall (bis zum Auftreten von Atemnotanfällen) der Kontrolle verlängert (Kruskal-Wallis-Test, n = 10).

Dagegen traten die Atemnotfälle der mit der Kombination RSH und Verapamil behandelten Meerschweinchen signifikant später auf als bei den Kontrollen. Dies ist als Schutzeffekt im Sinne einer Bronchospasmolyse zu interpretieren, die auf einer synergistischen Wirkung beider Substanzen beruht.

2. Untersuchung der Herzfrequenz-senkenden Wirkung an Meerschweinchen

Wachen Meerschweinchen wird über 45 Minuten 1 μg/kg/min Isoprenalin (1. Gruppe) oder 0,9 %-ige NaCl-Lösung (Kontroll-Gruppe) infundiert. Eine 3. Gruppe erhält bis zur 30. Minute Verapamil (250 μg/kg/min i.v.) und ab der 30. Minute bis zum Versuchsende (45. Minute) zusätzlich Isoprenalin (1 μg/kg/min i.v.). Während der gesamten Versuchsdauer wird die Herzfrequenz registriert.

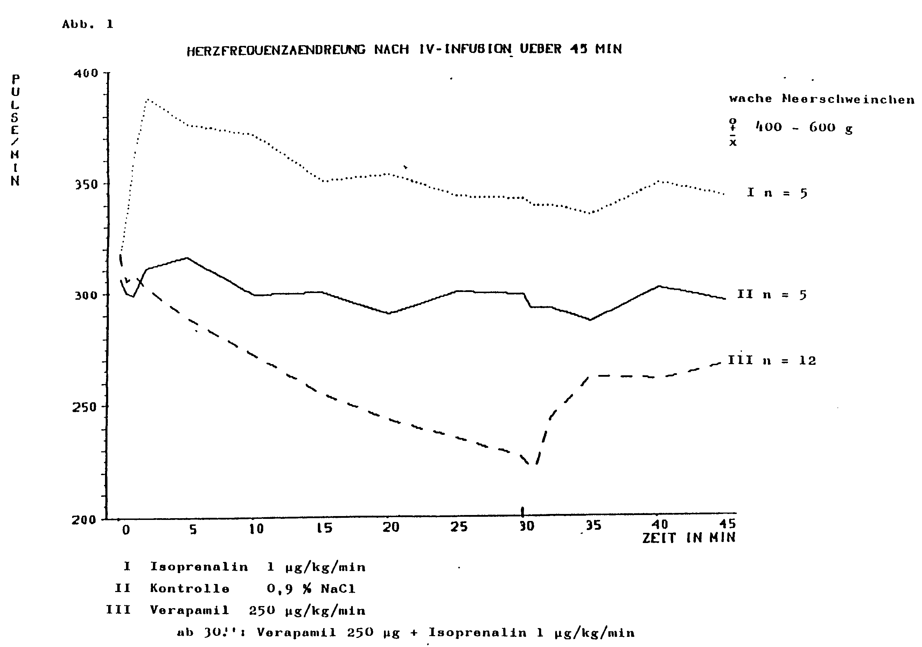

Das Ergebnis ist in Abb. 1 dargestellt.

Unter der Infusion von Isoprenalin steigt die Herzfrequenz von im Mittel ($\bar{x}$, n = 5) von 315 auf maximal 385 Schläge pro Minute (~ + 22 %) an, während die Herzfrequenz der Kontrollen (n = 5) annähernd konstant bleibt. Die Herzfrequenz der mit Verapamil behandelten Meerschweinchen (n = 12) fiel von einem Ausgangswert von im Mittel etwa 315 Schlägen/min. kontinuierlich ab und hatte 30 Minuten nach Infusionsbeginn den Wert von 220 pulsen/min. erreicht (~ - 30 %).

Bei zusätzlicher Infusion von Isoprenalin steigt von diesem Zeitpunkt an die Herzfrequenz bis zum Versuchsende auf 270 Pulse/min und liegt damit um etwa 22 % über dem 30-Minuten-Wert (Beginn der Isoprenalin-Infusion). Das Ergebnis bedeutet, daß die den β-Rezeptoren-Stimulatoren inhärente Herzfrequenzsteigernde Wirkung durch eine kombinierte Behandlung mit Verapamil dahingehend beeinflußt wird, daß die Herzfrequenz insgesamt im Normbereich bleibt.

3. Klinische Untersuchung der Herzfrequenz von Asthmatikern

Je 10 Asthmatiker wurden entweder oral mit Terbutalin (2 x 1 Tablette Bricanyl® duriles pro Tag) oder per Inhalation mit Terbutalin (initial 1 x 2 Hübe, danach 4 x 2 Hübe über den Tag verteilt) über 48 Stunden behandelt und erhielten in den folgenden 48 Stunden zusätzlich täglich 2 x 40 mg Verapamil (Isoptin®) per os.

Vor Behandlungsbeginn sowie 2, 24 und 48 Stunden nach der Terbutalingabe und 2, 24 sowie 48 Stunden nach der 1. Verapamil -Gabe wurden die Resistance (= Strömungswiderstand in den Hauptluftwegen) und die Herzfrequenz gemessen.

Die Ergebnisse der Untersuchung sind in den Tabellen II und III zusammengestellt. Bei oraler Behandlung mit der Kombination von Terbutalin und Verapamil war die Herzfrequenz zu allen Meßzeitpunkten (2 h, 24 h, 48 h) im Mittel niedriger als zu den entsprechenden Meßzeitpunkten nach alleiniger Gabe von Terbutalin.

Für die Behandlung mit der Kombination Terbutalin-Spray plus Verapamil oral ist der Herzfrequenz-senkende Effekt des Verapamils gegenüber dem Vorwert (= alleinige Terbutalinbehandlung) für den 24 h-Wert (p < 0,1) und dem 48 h-Wert (p < 0,05) statistisch gesichert (Kruskal-Wallis-Test).

**Tabelle II**

Einfluß von Terbutalin oral sowie Terbutalin oral plus Varapamil oral auf die Herzfrequenz und die Resistance von 10 Asthmatikern

**RESISTANCE** ($\frac{kPa}{1/sec}$)

| Behandlung | Vorwert | 2 x 1 Tablette Bricanyl® duriles/d | | | Bricanyl® + Isoptin® (2 x 40 mg/d p.o.) | | |
|---|---|---|---|---|---|---|---|
| | | 2 h | 24 h | 48 h | 2 h | 24 h | 48 h n. Behandl. |
| $\bar{x}$ | 8,47 | 6,84 | 6,84 | 6,83 | 6,50 | 6,28 | 6,19 |
| S$\bar{x}$ | 0,37 | 0,30 | 0,28 | 0,31 | 0,28 | 0,32 | 0,29 |

**HERZFREQUENZ** (Pulse/min)

| Behandlung | Vorwert | 2 x 1 Tablette Bricanyl® duriles/d | | | Bricanyl® + Isoptin® (2 x 40 mg/d p.o.) | | |
|---|---|---|---|---|---|---|---|
| | | 2 h | 24 h | 48 h | 2 h | 24 h | 48 h n. Behandl. |
| $\bar{x}$ | 76 | 75,2 | 75,0 | 74,2 | 68,2 | 67,8 | 66,6 |
| S$\bar{x}$ | 4,18 | 3,81 | 3,87 | 3,82 | 3,16 | 2,41 | 2,67 |

**Tabelle III**

Einfluß von Terbutalin-Spray sowie Terbutalin-Spray plus Verapamil oral auf die Herzfrequenz und die Resistance von 10 Asthmatikern

**RESISTANCE** ($\frac{kPa}{1/sec}$)

| Behandlung | Leerwert | 18 Hübe / 48 h Bricanyl®-Spray | | | Bricanyl®-Spray + Isoptin® (2 x 40 mg/d p.o.) | | |
|---|---|---|---|---|---|---|---|
| | | 2 h | 24 h | 48 h | 2 h | 24 h | 48 h n. Behandl |
| $\bar{x}$ | 11,99 | 6,58 | 8,47 | 8,43 | 8,14 | 8,18 | 8,31 |
| S$\bar{x}$ | 0,88 | 0,60 | 0,71 | 0,53 | 0,40 | 0,42 | 0,48 |

**HEZFREQUENZ** (Pulse/min)

| Behandlung | Leerwert | 18 Hübe / 48 h Bricanyl®-Spray | | | Bricanyl®-Spray + Isoptin® (2 + 40 mg/d p.o.) | | |
|---|---|---|---|---|---|---|---|
| | | 2 h | 24 h | 48 h | 2 h | 24 h | 48 h n. Behandl. |
| x̄ | 95,6 | 97,0 | 93,6 | 90,0 | 87,0 | 81,4 | 79,6 |
| Sx̄ | 3,45 | 3,19 | 3,78 | 2,92 | 3,27 | 4,32 | 4,38 |

Die erfindungsgemäße pharmazeutische Zusammensetzung ist somit in der Humanmedizin zur Behandlung des Asthma bronchiale geeignet. Sie enthält den verwendeten β-Rezeptor-Stimulator im Verhältnis zum Verapamil von 1 : 100 bis 1 : 1000 Gewichtsteilen.

Die erfindungsgemäße pharmazeutische Zusammensetzung wird in der Praxis analog den β-Rezeptor-Stimulatoren eingebis 100 mg liegt.

Die erfindungsgemäße pharmazeutische Zusammensetzung kann sowohl inhalativ als auch oral gegeben werden. Die tägliche Dosis liegt bei 50 mg bis 200 mg, vorzugsweise bei 100 mg bis 150 mg.

Für die orale Applikation kann die erfindungsgemäße Zusammensetzung z. B. als Tablette, Kapsel, Dragee, Granulat, Saft, Suspension oder Sirup vorliegen. Zu den Wirkstoffen können die in der Galenik üblicherweise verwendeten Trägersubstanzen, wie z. B. Talkum, kalloidales Siliciumdioxid, Stärke, Lactose und Magnesiumstearat sowie Netz-, Dispergier- oder Emulgiermittel und/oder Konservierungsmittel zugesetzt werden.

Für die inhalative Applikation wird die erfindungsgemäße pharmazeutische Zusammensetzung mit den üblichen Zusätzen in einem üblichen physiologisch verträglichen Treibgas, wie z. B. Frigen®, suspendiert und die so erhaltene Suspension in Sprühdosen, die vorzugsweise mit einem Dosierventil versehen sind, abgefüllt.

Für die Zerstäubung ist es vorteilhaft, die Wirkstoffe durch Mikronisieren auf eine mittlere Korngröße von 0,01 bis 20 µm zu bringen. Zur Erhöhung der Lagerfähigkeit und zur Erleichterung der Aerosolbildung kann man diesen Mischungen zweckmäßigerweise noch feste, pharmakologisch unwirksame wasserlösliche pulverförmige Feststoffe mit einer mittleren Korngröße von 20 bis 200 µm zusetzen. Als solche eignen sich beispielsweise Dextran, Mannit, Glyceride, Laktose und Sorbitansäureester wie Sorbitansesquioleat.

Die nachstehenden Beispiele sollen die erfindungsgemäße pharmazeutische Zusammensetzung erläutern.

**Beispiel 1**

**Tabletten**

| | |
|---|---|
| Verapamil | 50,00 mg |
| RSH | 0,05 mg |
| Lactose | 88,95 mg |
| Maisstärke | 50,00 mg |
| Gelatinierte Maisstärke | 8,00 mg |
| Calciumstearat | 3,00 mg |
| Totalgewicht | 200,00 mg |

Die Wirkstoffe, die Lactose, die Maisstärke und die gelatinierte Maisstärke werden innig miteinander vermischt. Die Mischung wird durch eine Zerkleinerungsmaschine passiert und anschließend mit Wasser zu einer dicken Paste befeuchtet. Die angefeuchtete Masse wird durch ein Sieb passiert. Das erhaltene Granulat wird bei 45° C getrocknet und mit dem Calciumstearat vermischt. Das Granulat wird zu Tabletten von einem Gesamtgewicht von 200 mg und einem Durchmesser von etwa 8 mm gepreßt.

**Beispiel 2**

**Tabletten**

**Aerosol**

Eine Sprühdose, die mit einem Dosierventil versehen ist, wird mit

| | |
|---|---|
| Verapamil | 200,00 mg |
| RSH | 0,20 mg |

| | | |
|---|---|---|
| Sorbitansequioleat | 0,30 mg | |
| Triglycerid (Miglyol® 812) | 120,00 mg | und |
| Frigen 113® | 760,00 mg | |

gefüllt. Die Menge des Dosier-Aerosols reicht für 200 Hübe à 50 µl Freigabe/Hub.

**Patentansprüche**

1. Pharmazeutische Zusammensetzung bestehend aus Verapamil und einem β-Rezeptor-Stimulator.

2. Pharmazeutische Zusammensetzung gemäß Anspruch 1, dadurch gekennzeichnet, daß der β-Rezeptor-Stimulator der 4-(2-tert.-Butylamino-1-hydroxyethyl-7-hydroxy-2-indolcarbonsäure-methylester ist.

3. Pharmazeutische Zusammensetzung gemäß Anspruch 1, dadurch gekennzeichnet, daß das Gewichtsverhältnis des β-Rezeptor-Stimulators zum Verapamil im Bereich von 1 : 100 bis 1 : 1000 liegt.

4. Pharmazeutische Zusammensetzung gemäß Anspruch 1 - 3, dadurch gekennzeichnet, daß die Zusammensetzung für die Applikation per inhalationem Trägersubstanzen enthält.

5. Pharmazeutische Zusammensetzung gemäß Anspruch 1 - 3, dadurch gekennzeichnet, daß die Zusammensetzung für die orale Applikation Trägersubstanzen enthält.

6. Verfahren zur Herstellung der pharmazeutischen Zusammensetzung gemäß den Ansprüchen 1 - 4, dadurch gekennzeichnet, daß man Verapamil und einen β-Rezeptor-Stimulator mit einer pharmazeutischen Trägersubstanz zu einem pharmazeutischen Präparat verarbeitet.

**Claims**

1. Pharmaceutical preparation comprising Verapamil and a β-receptor-stimulator.

2. Pharmaceutical preparation according to claim 1, characterised in that the β-receptor-stimulator is 4-(2-tert.-butylamino-1-hydroxyethyl-7-hydroxy-2-indolecarboxylic acid methyl ester.

3. Pharmaceutical preparation according to claim 1, characterised in that the weight ratio of the β-receptor-stimulator to Verapamil is in the range of from 1 : 100 to 1 : 1000.

4. Pharmaceutical preparation according to claims 1 to 3, characterised in that the preparation contains carrier substances for application by inhalation.

5. Pharmaceutical preparation according to claims 1 to 3, characterised in that the preparation contains carrier substances for oral administration.

6. Process for the manufacture of the pharmaceutical preparation according to claims 1 to 4, characterised in that Verapamil and a β-receptor-stimulator are processed with a pharmaceutical carrier substance to form a pharmaceutical preparation.

**Revendications**

1. Composition pharmaceutique constituée de vérapamil et d'un stimulant des récepteurs β.

2. Composition pharmaceutique selon la revendication 1, caractérisée en ce que le stimulant des récepteurs β est le (tert-butylamino-2 hydroxy-1 éthyl)-4 hydroxy-7 indole-carboxylate-2 de méthyle.

3. Composition pharmaceutique selon la revendication 1, caractérisée en ce que le rapport pondéral du stimulant des récepteurs β au vérapamil est situé dans l'intervalle allant de 1 : 100 à 1 : 1000.

4. Composition pharmaceutique selon l'une quelconque des revendications 1 à 3, caractérisée en ce que la composition contient des matières supports pour l'application par inhalation.

5. Composition pharmaceutique selon l'une quelconque des revendications 1 à 3, caractérisée en ce que la composition contient des matières support pour l'application par la voie orale.

6. Procédé de préparation de la composition pharmaceutique selon l'une quelconque des revendications 1 à 4, procédé caractérisé en ce qu'on travaille le vérapamil et un stimulant des récepteurs β avec une matière support pharmaceutique de manière à obtenir une composition pharmaceutique.

Abb. 1

HERZFREQUENZAENDREUNG NACH IV-INFUSION UEBER 45 MIN

P
U
L
S
E
/
M
I
N

wache Meerschweinchen

$\frac{\text{\textfemale}}{\bar{x}}$ 400 - 600 g

I n = 5

II n = 5

III n = 12

400

350

300

250

200

0   5   10   15   20   25   30   35   40   45
ZEIT IN MIN

I    Isoprenalin   1 µg/kg/min
II   Kontrolle     0,9 % NaCl
III  Verapamil    250 µg/kg/min
     ab 30.': Verapamil 250 µg + Isoprenalin 1 µg/kg/min

EP 0 201 571 B1